# EUROPEAN PATENT APPLICATION

(11) **EP 2 669 367 A1**
(43) Date of publication of application: **04.12.2013**
(21) Application number: 12170552.9
(22) Date of filing: 01.06.2012
(51) Int. Cl.: C12N 5/0784, A61K 39/00

(54) **A human dendritic cell line**

(71) Applicant: Stichting Katholieke Universiteit, 6525 GA Nijmegen (NL)
(72) Inventor: Van Leeuwen, Franciscus Nicolaas, 6531 NN Nijmegen (NL)
(74) Representative: Habets, Winand

(57) **Abstract**

The invention is in the field of medical tools and therapies. It provides a cell line and methods for its use. More in particular the invention provides the use of a HLA-A2 positive cell line, derived from human blood, with dendritic cell properties, its potential use as an agent or a vaccine for cancer immunotherapy and the treatment and/or prophylaxis of immune diseases. Furthermore the invention describes the use of these cells as a model system for studying the biology of dendritic cells or dendritic cell/pathogen interactions. More in particular, the invention relates to a human cell or cell line, obtainable from human peripheral blood, with the phenotype: CD3-, CD4-, CD7-, CD163-, CD14-, CD34-, CD56-, CD19+, CD79-, CD11b-, CD11C+, CD1a+, CD40+, CD80+, CD83+, CD86+, HLA-DR+, HLA-DQ+, HLA-ABC+, DC-SIGN-, CD206-, DEC205+, DECTIN-, BDCA2-.

## Description

### Field of the invention

The invention is in the field of medical tools and therapies. It provides a cell line and methods for its use. More in particular the invention provides the use of a HLA-A2 positive cell line, derived from human blood, with dendritic cell properties, its potential use as an agent or a vaccine for cancer immunotherapy and the treatment and/or prophylaxis of immune diseases. Furthermore the invention describes the use of these cells as a model system for studying the biology of dendritic cells or dendritic cell/pathogen interactions.

### Background of the invention

Dendritic cells (DCs) are highly specialized antigen presenting cells (APC) that link innate and adaptive immune responses. As dendritic cells are uniquely capable of activating naïve T cells and orchestrate a wide variety of other immune responses, DCs are considered primary targets for vaccine development in the treatment of infectious diseases as well as cancer.

Conversely, DCs can be used to induce tolerance in the treatment of allergy, autoimmune diseases or transplant rejection. Although laboratories worldwide are exploring the therapeutic applications of DCs, many cell biological aspects of DC biology are still poorly understood. This is in part due to the fact that studying human DC-biology in vitro is difficult. For instance, DCs isolated from peripheral tissues such as human blood or skin are low in number, post-mitotic and have a lifespan of only a few days. For example, myeloid DCs, which can be isolated from the blood, make up less than 1% of the mononuclear fraction. Larger numbers of DCs can be derived by in vitro differentiation of bone marrow-derived CD34+ stem cells, peripheral blood mononuclear cells or monocytes.

Isolation of these DC-precursors from the bone marrow or blood is laborious whereas their subsequent differentiation towards DCs is time consuming (5-7 days) and requires the use of expensive cytokines (IL-4 and GM-CSF). Moreover, manipulation of gene expression, for example by transfection, retroviral transduction or electroporation is generally ineffective and associated with considerable cell death. Finally, cell biological experiments using these human-derived primary cells are often complicated due to inherent genetic and environmental variability between donors.

The use of human cell lines may overcome such problems, provided these cell lines show properties comparable to primary blood-derived (myeloid) DCs or DCs generated ex-vivo. A number of myeloid leukemia cell lines have been explored for this purpose. These cell lines may be at least partially differentiated towards a DC like phenotype, either by the use of cytokine cocktails or the combined use of protein kinase C (PKC) and Ca2+-mobilizing agents (Santegoets et al., J.Leukoc.Biol. 2008;84:1364-1373; van Helden et al. Immunol.Lett. 2008;117:191-197).

Although some of these cell models are useful for studying specific aspects of DC biology, none of these models generate homogeneous cell populations that recapitulate the most important aspects of DC biology.

The acute myeloid leukemia-derived cell MUTZ-3 is widely used as a human DC model. MUTZ-3 is not a cell line in the sense that it consists of a mixed population of CD34+ / CD14- and CD34- / CD14+ cells, which, by use of a cytokine cocktail including GM-CSF and IL-4, can either be differentiated towards a langerin positive Langerhans-like cell type or a DC-SIGN positive interstitial DC like phenotype. Importantly these cells were shown to posses functional antigen processing and presentation pathways, and were found capable of generating specific virus-reactive or tumor-reactive cytotoxic T cells (Masterson et al., Blood 2002;100:701-703; Santegoets et al., J.Leukoc.Biol. 2008;84:1364-1373). MUTZ-3 may therefore be used to generate DC-like cells capable of performing a number of DC cell functions.

A limitation of this model however, is the fact that MUTZ-3 derived DCs show a failure to respond to pathogen associated molecular patterns (PAMPS) (Rasaiyaah et al., Immunology 2009;127:429-441), a key feature of primary dendritic cells and an essential aspect of DC biology. Hence there is a continued need for human model-cell lines capable of performing other functions of DC cells, such as antigen uptake, antigen processing and presentation and that can accurately respond to PAMPS, for instance by activation through toll-like receptors (TLR) or stimulation with maturation-inducing cytokines. Such uniform DC cell line models may be used to facilitate the optimization and quality control of novel treatment modalities, allowing more rapid translation of such protocols into clinical practice. Moreover, they may be used in the therapy of cancer and the treatment and/or prophylaxis of immune diseases.

### Summary of the invention

The present invention therefore relates to a novel human cell line, obtainable from human peripheral blood, tentatively named TDC2. This cell line both phenotypically and functionally resembles DCs present in the blood (mDCs) and is of the following phenotype: CD3-, CD4-, CD7-, CD163-, CD14-, CD34-, CD56-, CD19+, CD79-, CD11b-, CD11C+, CD1 a+, CD40+, CD80+, CD83+, CD86+, HLA-DR+, HLA-DQ+, HLA-ABC+, DC-SIGN-, CD206-, DEC205+, DECTIN-, BDCA2-.

The cell line designated herein as TDC2 has been deposited at the Deutsche Sammlung von Mikroorganismen und Zellkulturen GmbH (DSMZ or German Collection of Microorganisms and Cell Cultures) at Inhoffenstraße 7 B, 38124 Braunschweig, Germany under accession number DSM ACC3168 on 28.03.2012.

### Legend to the figures

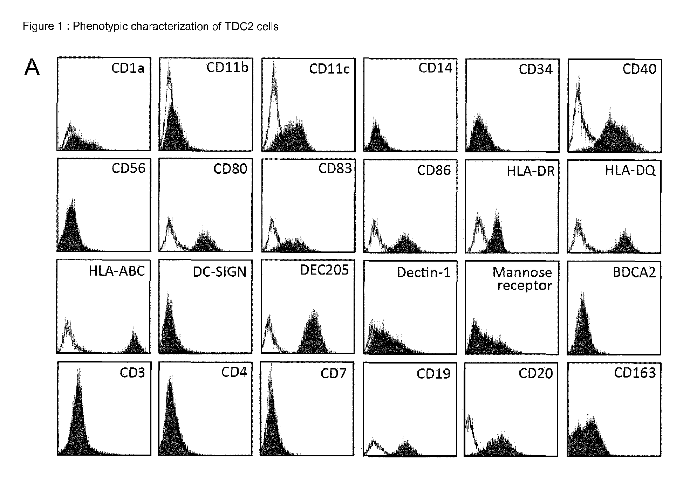

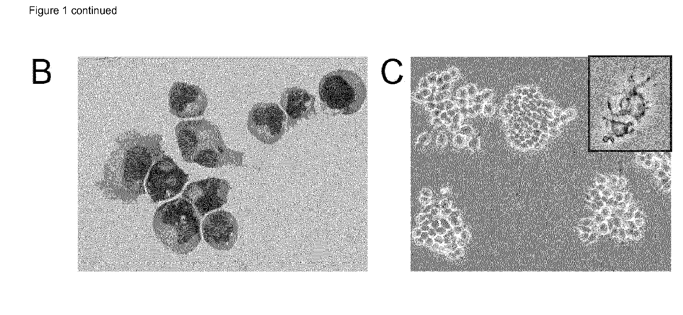

### Figure 1: Phenotypic characterization of TDC2 cells

(A) TDC2 cells display DC characteristics as determined by flow cytometry . Cells were stained with a large panel of antibodies reactive with immune-cell surface markers. Filled histograms show staining of specific antibodies and open histograms show staining of isotype-matched control antibodies For a complete list of cell surface proteins analyzed it is referred to table 1
(B) May-Grunwald-Giemsa stained cytocentrifuge preparations of TDC2 cells reveal a DC/macrophage like morphology.
(C) Clusters of TDC2 cells grown in suspension cultures determined by phase contrast microscopy

### Figure 2: TDC2 cells express a broad repertoire of TLRs

TLR expression was analyzed by real-time quantitative PCR. See Table 2 for primer sequences. TLR expression is shown relative to expression in monocyte-derived DCs.

### Figure 3 : TDC2 cells efficiently take up particulate and soluble antigens

TDC2 cells, seeded on poly-lysine coated coverslips, were incubated for 30, 60 and 120 minutes with dextran-coated iron particles (endorem) at 37 °C, fixed and subjected to prussian blue staining to allow detection of internalized iron particles. Positive cells were scored by microscopic inspection. As a control cells were kept on ice during incubation. Data are shown as mean ± SEM of 7 independent experiments. *** p<0.001 (two-way ANOVA followed by Bonferroni posttest).

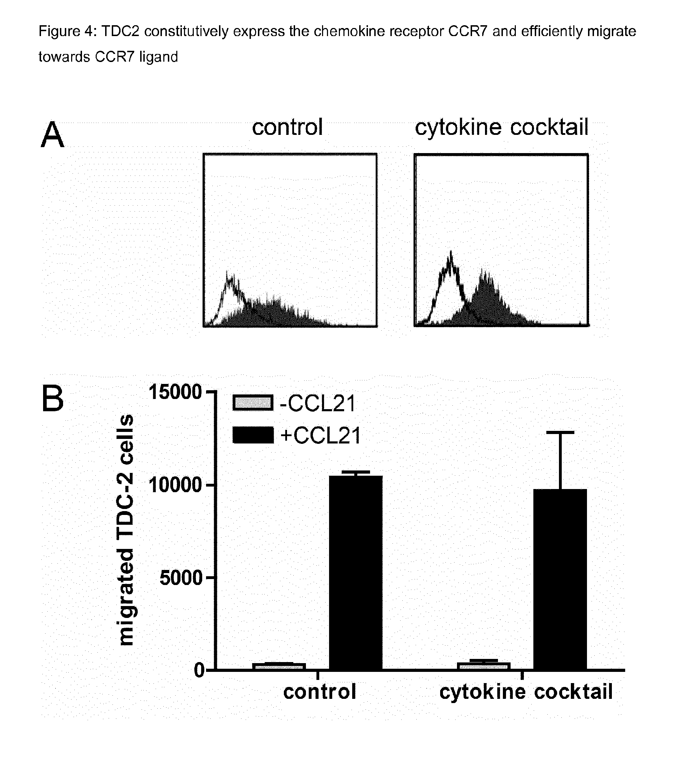

### Figure 4: TDC2 constitutively express the chemokine receptor CCR7 and efficiently migrate towards CCR7 ligand

(A) Expression of CCR7 was analyzed by flow cytometry on unstimulated TDC2 cells and TDC2 cells stimulated for 48 hours with a cytokine cocktail consisting of IL-1β, IL-6, TNFα and PGE₂. Filled histograms represent TDC2 cells stained with anti-human CCR7 antibodies and open histograms represent matched isotype controls.
(B) CCR7-mediated migration of unstimulated TDC2 cells and TDC2 cells stimulated for 48 hours with a cytokine cocktail. Migration was determined by the number of TDC2 cells that had migrated into the lower compartment of a transwell system containing 100 ng/ml CCL21. To measure spontaneous migration, cells were incubated in a transwell chemotaxis assay without CCL21 in the upper and lower compartment. The graph shows mean ± SD of a representative experiment performed in duplicate.

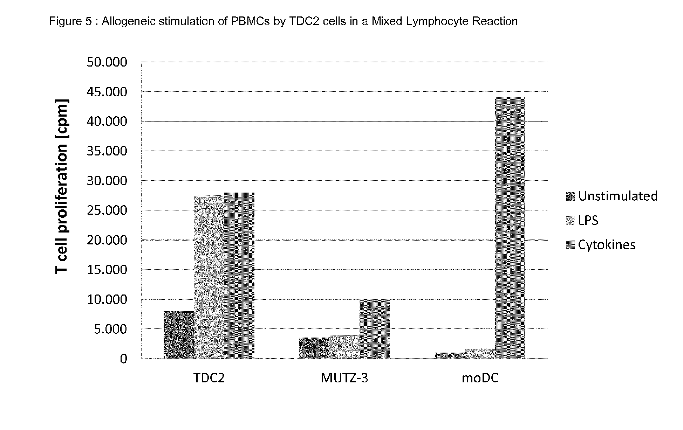

### Figure 5: Allogeneic stimulation of PBMCs by TDC2 cells in a Mixed Lymphocyte Reaction

The allostimulatory capacity of TDC2 cells was tested in a mixed lymphocyte reaction (MLR). 1x10⁵ allogeneic PBMCs were cocultured with 1x10⁴ TDC2 cells, MUTZ-3 cells or monocyte-derived DCs (moDCs), either unstimulated or stimulated with either LPS or cytokine cocktail for 48 hours, for four days and T cell proliferation was measured by incorporation of tritiated thymidine. The figure show a representative experiment out of three experiments performed. No T cell proliferation was observed with PBMC only or with DC in the absence of PBMC.

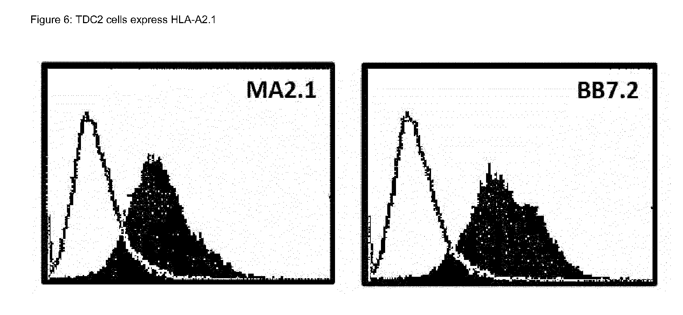

### Figure 6. TDC2 cells express HLA-A2.1

HLA-A2 expression on TDC2 cells was analyzed by flow cytometry with two independent antibodies (resp. MAC2.1 and BB7.2). Filled histograms represent TDC2 cells stained with specific antibodies and open histograms represent matched isotype control antibodies.

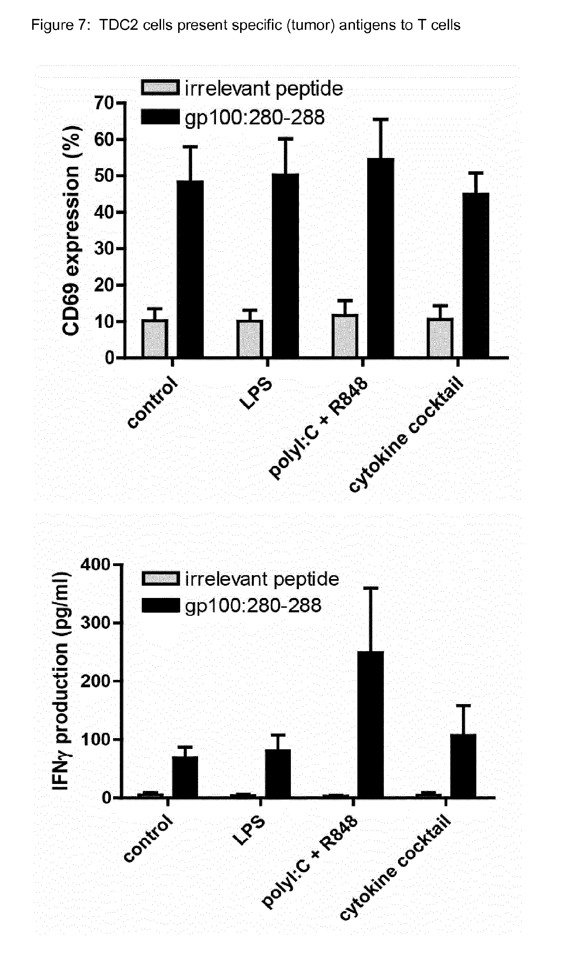

### Figure 7: TDC2 cells present specific (tumor) antigens to T cells

To study antigen-specific T cell activation, peripheral blood lymphocytes (PBLs) specific for gp100:280-288 (50.000 per well) were co-cultured with unstimulated TDC2 cells or TDC2 cells stimulated with either LPS, poly(I:C)+R848, or a cytokine cocktail (5.000 per well) that were loaded with either gp100:280-288 or an irrelevant peptide. After 16 hours antigen-specific activation of CD8⁺ T cells was analyzed by measurement of CD69 surface expression on CD8+ T cells (left panel) and secretion of IFNγ in the supernatant (right panel). The figure shows mean ± SEM of three independent experiments performed in duplicate.

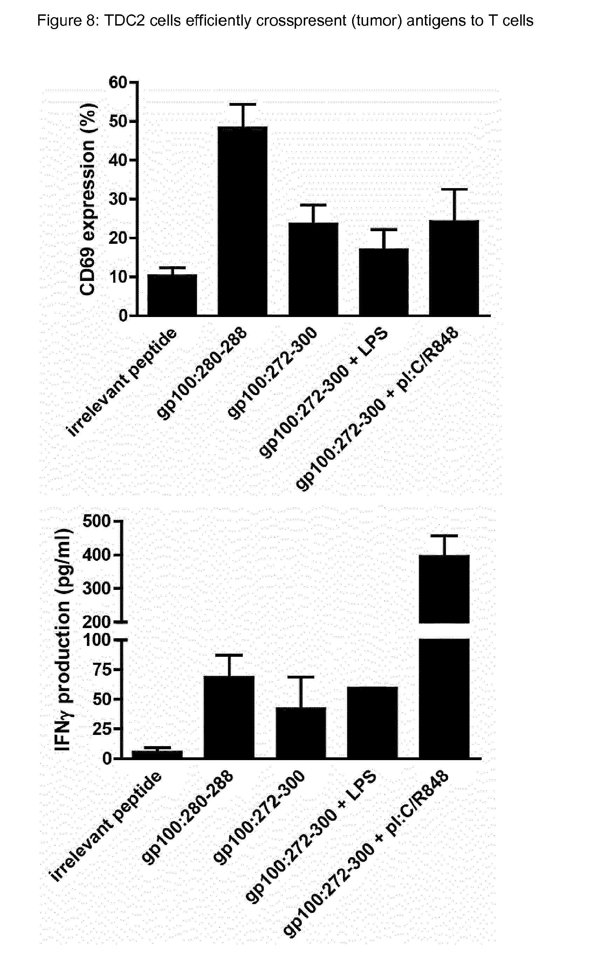

### Figure 8: TDC2 cells efficiently cross present tumor antigens to CD8+ T cells.

To study cross-presentation, TDC2 cells were loaded with gp100:272-300 long peptide for four hours. Gp100:280-288 short peptide and an irrelevant peptide served as a positive and negative control, respectively. Next, antigen-loaded TDC2 cells were co-cultured with PBLs specific for gp100:280-288 in the presence or absence of LPS or poly(I:C)+R848. After 16 hours antigen-specific activation of CD8⁺ T cells was analyzed by measurement of CD69 surface expression on CD8+ T cells (left panel) and secretion of IFNγ in the supernatant (right panel). The figure shows mean ± SEM of three independent experiments performed in duplicate.

### Detailed description of the invention

Dendritic cells (DCs) are highly specialized antigen presenting cells that are the primary targets for vaccine development in the treatment of infectious diseases and cancer. Due to low frequencies and short lifespan of naturally occurring DCs and laborious culture conditions of in vitro differentiated DCs, it is difficult to study these cells ex vivo. As a consequence, many aspects of DC biology are still poorly understood while practical human preclinical models are lacking. The use of standardized human DC cell lines with consistent characteristics and high purity would overcome such problems.

Herein, we describe the isolation and characteristics of a cell line derived from the blood of a patient with T-cell acute lymphoblastic leukemia (T-ALL), which is unrelated to the patient's leukemia. This highly uniform cell line, named TDC2 (T-ALL derived Dendritic Cell line-2), displays many phenotypic and functional characteristics of DCs, most notably efficient antigen uptake, processing and cross-presentation. In addition, TDC2 cells express a broad repertoire of TLRs and can be activated by either cytokine stimulation or TLR ligands. Hence TDC2 represents an attractive new model for studying DC biology and may be useful as platform to accelerate the clinical application of DC vaccines.

Human myeloid DCs (mDCs), also known as conventional DCs (cDCs), represent a subset of DCs present in circulating blood, which can be identified by means of their morphological and functional characteristics, combined with expression of specific cell surface antigens/receptors. In the blood, distinct subsets of mDCs can be recognized showing distinct marker expression patterns, although there is a considerable heterogeneity in expression of these mDC markers. Cell surface phenotype analyses are usually carried out by flow cytometry. Human mDCs in particular express MHC class I and class II molecules, costimulatory molecules (CD80, CD83, CD860) as well as the lectin DEC205. The cell surface markers CD11 c, DC-SIGN, CD1 a, and BDCA1 can be found on some but not all mDC subsets.

In order to obtain TDC2, PBMCs were isolated from the blood of a 2 year old boy diagnosed with T-ALL and maintained in culture for several weeks. Leukemic blasts, obtained at diagnosis, carried a (11;14)(p13;q11)translocation, which occurs in about 7% of all T-ALL cases (Hwang and Baer., Curr.Opin.lmmunol. 1995;7:659-664). After a few weeks of culturing, loosely-adherent cell clusters grew out, giving rise to a cell type clearly distinct from the original leukemic blast population. Cytogenetic analysis revealed a normal karyotype , indicating that this cell population was unrelated to the T-ALL. Consistent with these observations, we determined by flow cytometry that the T cell markers CD3, CD4, CD7, used to define the leukemic blast population, were not present on these cells (Figure 1A).

Further flow cytometric analysis identified these cells as dendritic cell-like, as indicated by the prominent expression MHC class II molecules (HLA-DR/HLA-DQ), MHC class I (HLA-ABC), DEC205, CD1 a, CD11c, and the expression of costimulatory molecules CD80, CD86 and CD83. In addition, the early B cell markers CD19 and CD20 were found to be present on these cells. The cells also expressed the Fc receptors CD23 (FcεRII), CD32 (FcγRIII) and CD16 (FcγRII) and C type lectin receptors dectin-1 and mannose receptor, whereas DC markers BDCA1, BDCA2, BDCA3 and BDCA4, C type lectin receptors DC-SIGN, DCIR and CLEC9A, and the macrophage marker CD163 were absent. Table 1 shows a complete overview of cell surface markers analyzed.

**Table 1. Schematic overview cell surface molecules analyzed on TDC2 cells**

| | **Receptor** | **Expressed** |
|---|---|---|
| **Plasmacytoid DCs** | BDCA-2 | - |
| | BDCA-2 | - |
| | CD123 | + |
| **Myeloid DCs** | BDCA1 | - |
| | BDCA3 | - |
| | CLEC9A | - |
| **Fc receptors** | CD16 | - |
| | CD23 | + |
| | CD32 | + |
| **lntegrins** | CD11b | - |
| | CD11c | + |
| **C-type Lectins** | Mannose receptor | - |
| | DC-SIGN | - |
| | DCIR | - |
| | Dectin-1 | - |
| | DEC-205 | + |
| **Chemokine receptor** | CCR7 | + |
| **Antigen presentation** | HLA-ABC | + |
| | HLA-DR/DQ/DP | + |
| | CD1a | +/- |
| **Costimulatory molecules** | CD40 | + |
| | CD80 | + |
| | CD86 | + |
| **DC maturation** | CD83 | + |
| **T-cell inhibition** | PD-L1 | + |
| | PD-L2 | + |
| **T-Lymphocytes** | CD3 | - |
| | CD4 | - |
| | CD7 | - |
| **B-lymphocytes** | CD19 | + |
| | CD20 | + |
| **Monocytes** | CD14 | - |
| **Macrophages** | CD163 | - |
| **NK cells** | CD56 | - |

The same cell population was recovered repeatedly, from either blood or bone marrow obtained from this patient while in complete remission. Again, after a few weeks of culturing, highly proliferating cell clusters were acquired that could be maintained for at least 100 passages. In May-Grünwald/Giemsa stained cytocentrifuge preparations, the cells were characterized as large (sometimes binucleate) cells with large cytoplasms. Plasmamembrane extensions and vacuolizations were frequently observed, a morphology indicative of DCs. (Figure 1 B).

In the culture dish, the cells grew as large aggregates with fine membrane extensions (Figure 1 C). At higher magnifications, we observed that the fraction of the cells that had efficiently adhered to the substrate carried prominent finger-like projections, characteristic of DCs. When the cells were allowed to adhere to fibronectin/poly-I lysine-coated coverslips and stained for F-actin, a typical DC morphology was revealed, evidenced by prominent actin-rich dendritic extensions. Hence, based on cell surface expression and morphological criteria, these blood-derived cells may represent a class of (poorly adherent) myeloid or conventional DCs.

These cells were termed TDC2 cells and deposited at the Deutsche Sammlung von Mikroorganismen und Zellkulturen GmbH (DSMZ or German Collection of Microorganisms and Cell Cultures) at Inhoffenstraße 7 B, 38124 Braunschweig, Germany under accession number DSM ACC3168 on 28.03.2012.

Hence, the invention relates to a human cell or cell line as deposited at the DSMZ under accession number DSM ACC3168. This cell line has the phenotype: CD3-, CD4-, CD7-, CD163-, CD14-, CD34-, CD56-, CD19+, CD79-, CD11b-, CD11C+, CD1 a+, CD40+, CD80+, CD83+, CD86+, HLA-DR+, HLA-DQ+, HLA-ABC+, DC-SIGN-, CD206-, DEC205+, DECTIN-, BDCA2-.

The term cell line applies to mammalian cells that show proliferative capacity in culture. Preferably this proliferative capacity lasts for a number of passages such as 10 passages or more, such as 20, 30, 40, 50, 60, 70, 80 or 90 or more passages such as more than 100 passages. Most preferred is an infinite or unlimited capacity to proliferate. Primary DC cultures, obtained from peripheral blood or otherwise, do not multiply. The cell line according to the present invention is capable of multiplying for at least 100 divisions in culture, making it possible to obtain large amounts of these cells in vitro. The doubling time is about 72 hrs. Hence TDC2 cells can be considered immortal as they appear to multiply indefinitely in vitro.

Although the initiation of effective antigen-specific immunity (or tolerance to self-antigens) is a primary feature of DCs, these cells also play a central role in the control of innate immune responses to a large variety of pathogens. To do so, DCs are equipped with a broad repertoire of receptors involved in pathogen recognition, in particular Toll like receptors (TLRs) (Iwasaki and Medzhitov. Science 2010;327:291-295).

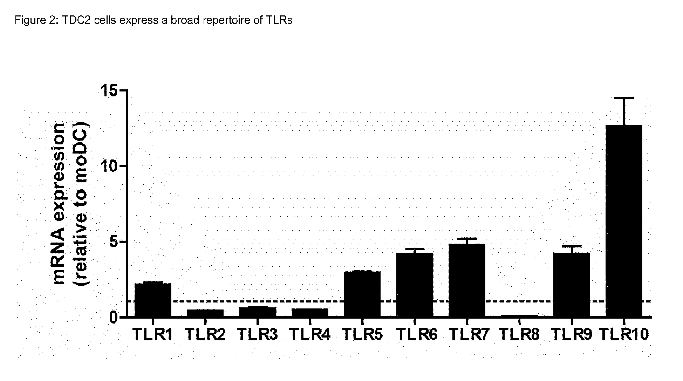

To date, 10 different members of the TLR family have been identified, and their primary function is to sense and respond to pathogen-associated molecular patters form bacteria, viruses or parasites. Stimulation TLRs, by pathogens, provides essential stimulatory signals for DC maturation and the initiation of adaptive immune responses against these pathogens. In addition TLRs can recognize endogenous ligands (self-antigens) to initiate inflammatory responses and tissue repair without triggering adaptive immune responses (reviewed by Iwasaki and Medzhitov. Science 2010;327:291-295). Although DCs express most TLRs, different DC subsets show distinct TLR expression profiles (Schreibelt et al, Cancer Immunol.Immunother. 2010;59:1573-1582). For instance, human circulating myeloid DCs express all TLRs, with the exception of TLR9, whereas plasmacytoid DCs express high levels of TLR7 and TLR9, intracellular receptors that are triggered by viral DNA or RNA, leading to the secretion of high amounts of Type I IFN. Based on mRNA expression, TDC2 cells resemble monocyte-derived DCs (moDCs) and myeloid DCs, however, in contrast to most myeloid DC subsets characterized to date, the TDC2 cells appear to also express TLR7 and TLR9, while TLR8 expression is lacking (Figure 2). Thus, the TLR expression profile of TDC2 cells appears to be a mixture of that of myeloid and plasmacytoid DCs.

One of the defining properties of DCs and other antigen presenting cells is their ability to take up soluble or particulate antigens and process these for antigen presentation in the context of MHC I or MHC II molecules. This can occur via receptor-mediated endocytosis, phagocytosis or macropinocytosis (fluid phase uptake) (Lanzavecchia Curr.Opin.Immunol. 1996;8:348-354).

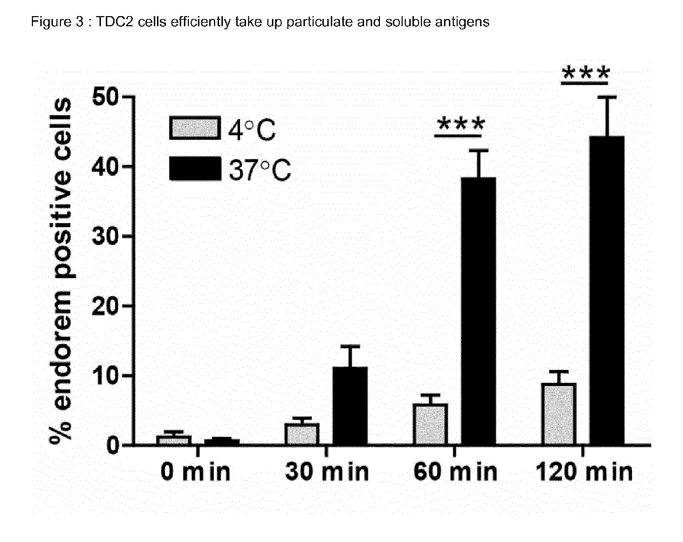

Hence, we tested the ability of TDC2 cells to take up and process particulate or soluble antigens. Hereto, TDC2 cells, seeded on coverslips, were incubated for 30, 60 and 120 minutes with dextran-coated iron particles (endorem) both at 37 °C and at 4 °C (to block membrane traffic), fixed and subjected to Prussian blue staining, to allow detection of internalized iron particles. Positive cells were scored by microscopic inspection (Figure 3). We observed that after two hours of incubation at the physiological temperature of 37 °C, about 50% of the cells had internalized endorem particles, whereas less than 10% of the cells stained positive after incubation at 4 °C (Figure 3). Uptake of particles was not affected by LPS stimulation.

Similarly, TDC2 cells were seeded on poly-lysine coated coverslips and incubated with FITC-labelled ovalbumin (FITC-OVA, green) for 5 hrs at 37 °C, fixed, and stained with the lysosomal marker LAMP-1 (red). We determined that TDC2 cells incubated with FITC-labelled ovalbumin (FITC-OVA) had efficiently taken up and processed soluble antigens, ending up in a LAMP-1 positive compartment as determined by immunofluorescence microscopy. Hence, TDC2 cells efficiently take up soluble and particulate antigens.

Dendritic cell (DC) homing to the lymphatics and lymph nodes is essential for the induction of adaptive immune responses, and regulated by gradients of CCL19 and CCL21, ligands for the C-C chemokine-receptor CCR7. Upon activation or maturation, DCs upregulate CCR7, which allows them to sense and home towards CCR7 ligand-secreting lymph nodes (Förster et al, Nat Rev Immunol, 2008: 362-71).

Consistent with the mature nature of the TDC2 cells, expression of CCR7 was readily detected on TDC2 cells while not significantly affected by 48 hr incubation with the cytokine cocktail (Figure 4A). We next tested the ability of TDC2 cells to migrate towards a CCL21 gradient, using a transwell DC chemotaxis assay. Indeed, TDC2 cells efficiently migrated in a CCL21-dependent manner, even without prior stimulation with the cytokine cocktail (Figure 4B). Hence it is concluded that TDC2 efficiently migrate towards CCR ligands and that this chemokine-directed migration does not require prior stimulation with a cytokine maturation cocktail.

An important functional characteristic of DCs is their ability to effectively present antigens and stimulate T cell proliferation. In order to determine to what extent TDC2 cells were capable of inducing lymphocyte proliferation, an allogeneic mixed lymphocyte reaction (MLR) was performed in which we compared TDC2 cells with MUTZ-3 derived DCs (M3DCs) as well as monocyte-derived DCs (MoDCs). Freshly isolated allogeneic peripheral blood mononuclear cells (PBMCs or peripheral blood lymphocytes PBL) were isolated from two healthy donors and incubated with TDC2 cells in various effector/responder ratios. For these experiments, cells were either left unstimulated or primed with either LPS or a cytokine cocktail. Already in the absence of maturation stimuli, the TDC2 cells were able to induce some lymphocyte proliferation, consistent with relatively high expression of MHC and costimulatory molecules on these cells (Figure 5). However, lymphocyte proliferation could be further enhanced by prior exposure of the TDC2 cells to either LPS or the cytokine cocktail, indicating that these maturation stimuli trigger a robust activation of the antigen presentation machinery in these cells.

In contrast to TDC2 cells, (immature) MUTZ3-derived DCs and for this donor also the (immature) MoDCs were completely unresponsive to LPS treatment. Only after incubation with the maturation cocktail, effective stimulation of lymphocyte proliferation was seen with the MoDCs, while only a moderate effect was observed with the MUTZ3-derived DCs. These results demonstrate that TDC2 cells show strong allostimulatory capacity, which can be further enhanced in response to exposure with LPS or proinflammatory cytokines.

The large majority of tumor specific peptides available to the clinic to monitor tumor antigen-specific cytotoxic T cell responses, are presented in the context of HLA-A2. We determined by flow cytometry using anti-HLA-A2 monoclonal antibodies, BB7.2 and MA2.1, that TDC2 cells are HLA-A2 positive (Figure 6). This allowed us to monitor the induction of (tumor) antigen-specific T cell responses by TDC2 cells.

To monitor peptide-specific lymphocyte activation, PBLs expressing a specific T cell receptor (TCR) which recognizes an antigenic peptide derived from the melanoma specific antigen glycoprotein 100, (gp100:280-288) in the context of HLA-A2.1, were generated as described earlier (Schreibelt et al., Blood 2010;116:564-574). HLA-A2.1 positive TDC2 cells were either left untreated, stimulated with TLR-agonists or a cocktail of proinflammatory cytokines for 48 hrs prior to peptide loading. Subsequently, TDC2 cells were loaded with the gp100:280-288 short peptide, which can directly bind to extracellular membrane-bound HLA-A2.1. As a negative control, TDC2 cells were incubated with an irrelevant peptide (tyrosinase). After peptide-loading, TDC2 cells were co-cultured overnight with allogeneic HLA-A2.1 negative PBLs, made to express the T cell receptor that recognizes the HLA-A2.1-binding gp100:280-288 peptide. TDC2 cells induced gp100:280-288 peptide-specific T cells activation, as demonstrated by CD69 expression and IFNγ production after overnight co-incubation of TDC2 cells and gp100-specfic T cells (figure 7). Interestingly, T cells activated by poly(I:C) and R848-stimulated TDC2 cells produced higher levels of IFNγ than T cells activated by unstimulated TDC2 cells, suggesting that these TLR ligands induce further TDC2 activation. These data show that TDC2 cells can activate CD8+ T cells in an antigen-specific manner.

DCs can present antigens to CD8+ T cells either by endogenous processing of antigens (for instance after viral infection) or by the uptake and presentation of exogenous antigens, a phenomenon known as cross-presentation. While both modes of antigen presentation are probably important for mounting an immune response to infectious diseases, in the case of anti tumor responses, the generation of a CD8+ T cells response to intact tumor antigens (for instance derived from apoptotic or necrotic cells), is dependent on efficient cross-presentation (Melief CJ. Immunity. 2008;29:372-383). To date, very few DC model cell lines were shown to be capable of effective cross-presentation. We tested the ability of TDC2 cells to cross-present extracellular antigens to the gp100 specific T-lymphocytes. To this end, unstimulated TDC2 cells were incubated with either an irrelevant peptide (tyrosinase) or a gp100-derived long peptide (gp100:273-300) that requires active uptake and processing before it can be loaded onto HLA-A2.1. As a positive control, TDC2 cells were also loaded with the gp100:280-288 short peptide, which can directly bind to extracellular membrane-bound HLA-A2.1. After peptide incubation, TDC2 cells were either left untreated or stimulated with the TLR-4 ligand LPS or a combination of the TLR-3 agonist poly I:C and the TLR7/8 agonist R848 and cocultured overnight with the gp100:280-288 specific PBLs. TDC2 cells loaded with soluble gp100 long peptide induced significant T cell activation in terms of expression of the early activation marker CD69 and production of IFNγ, indicating that TDC2 cells efficiently cross-presented gp100 peptides to CD8+ T cells (Figure 8).

Moreover, prior stimulation of the TDC2 cells with the TLR ligands poly(I:C) and R848 markedly increased IFNγ production by gp100-specific T cells. We conclude from these experiments that TDC2 cells can effectively cross-present which can be further enhanced by TLR stimulation, making this cell line unique in its kind.

In summary, the invention relates to a human cell or cell line as deposited at the DSMZ under accession number DSM ACC3168. This cell line has the phenotype: CD3-, CD4-, CD7-, CD163-, CD14-, CD34-, CD56-, CD19+, CD79-, CD11b-, CD11C+, CD1 a+, CD40+, CD80+, CD83+, CD86+, HLA-DR+, HLA-DQ+, HLA-ABC+, DC-SIGN-, CD206-, DEC205+, DECTIN-, BDCA2-.

A cell line according to the invention may advantageously be used for a number of applications. For instance, the cell or cell line may be provided with an additional gene encoding an immunotherapeutic agent. Such a cell may then be used in the treatment of diseases.

According to the invention, introduction of genes is understood to be transfection or viral infection or transformation of cells or cell lines, thereby introducing genetic material into the cell or cell lines according to per se known methods. The genetic material can be DNA or RNA. The genetic material codes for the expression of at least one protein or peptide, or/and the RNA itself can have an inhibitory or stimulatory effect, e.g. as an antisense RNA. The proteins being expressed can be further processed and modified, e.g. by glycosylation. Genes can also be introduced by fusing cells or cell lines with other cells or cell lines.

According to the invention, immunotherapeutic agents are proteins and/or peptides which play a role in the use of the effective dendritic cells as immunotherapeutic agents. Suitable examples are e.g. tumor antigens, viral antigens or antigens from parasites, bacteria or other microorganisms. Cells or cell lines having immunotherapeutic agent genes incorporated therein will express the proteins or peptides of these genes, and these are presented to the immune system by the dendritic cells, so that the effective dendritic cells activate, inhibit or modulate corresponding immune responses, depending on the activity and effector stages of the effective dendritic cells. For presentation of the gene products, the expressed proteins or peptides are processed or directly used; furthermore, the expressed proteins or peptides can be modified, e.g. by glycosylation.

The invention also relates to mature dendritic cells obtainable by exposing the human cells or cell line according to claims 1 - 3 to at least one maturation stimulus. A skilled person will be aware of the myriad of stimulatory molecules available in the art suitable for the in vitro or in vivo maturation of dendritic cells.

The cell or cell line according to the invention was found capable of efficiently and effectively processing and/or presenting antigens. Hence, the invention relates to a human cell or cell line obtainable by exposing the human cell or cell line according to claims 1 -4 to an antigen and allowing the cell to process the antigen.

The cells or cell lines according to the invention may be used to manufacture advantageous pharmaceutical compositions or vaccines comprising the human cell or cell line of claims 1 -5 and a suitable excipient. Compositions comprising the cell or cell line according to the invention may advantageously be used as a medicament for the treatment of diseases wherein it is required to modulate the immune system, such as stimulating, inhibiting or activating the immune system.

Compositions comprising the human cell or cell line according to the invention may be advantageously used in the treatment or prophylaxis of an immune disease or cancer. In a preferred embodiment, the immune disease is selected from the group consisting of inflammatory diseases, autoimmune diseases, transplant rejections, and infectious diseases.

The cell or cell line according to the invention may also be used for investigational purposes. The invention also relates to a method for stimulating T-cells in vitro wherein the human cells or cell line according to the invention are exposed to T-cells, for example tumor antigen specific T cells.

The invention also relates to a method for identifying compounds that activate or inhibit a human dendritic cell function. For that purpose, the human cells or cell line according to the invention is exposed to a compound and the effect of the compound on the functional activation or inhibition of the cell function is determined.

In yet another embodiment of the invention, a method is provided for modulating, activating, inhibiting or stimulating the humoral and/or cellular immune system wherein a cell or cell line according to the invention is administered to an individual in need of such a treatment.

In yet another embodiment, the invention provides a method for efficiently presenting an antigen to the immune system wherein the antigen is contacted with the human cells or cell line according to the invention, incubated to allow the human cells or cell line to process the antigens and wherein the thus obtained cells are contacted with immune cells such as T cells or B cells. Such antigens may advantageously be selected from the group consisting of peptides, proteins, lipids, lipopeptides, lipoproteins, carbohydrates, glycolipids, glycopeptides, glycoproteins, phosphorylated proteins and phosphorylated peptides.

### Examples

### Example 1: Generation and maintenance of TDC2, a human blood derived cell line with DC like properties

PBMCs were isolated from the blood of a patient diagnosed with T-ALL by Ficoll density gradient separation and seeded 6-well culture plates (Costar Cambridge, MA). Cells were maintained in Memα supplemented with 20% FCS, 10% culture supernatant of a 5637 bladder carcinoma cell line (Quentmeier et al, . Leuk.Res. 1997;21:343-350), 100 U/ml penicillin/streptomycin solution and 50µM β-mercaptoethanol, unless stated otherwise. Cultures were fed with fresh medium every 5-7 days. After 4 weeks of culturing, large cell clusters grew out that had acquired a DC-like phenotype. Upon further culture, the cells were continuously growing and could be passaged once every three days. This continuously growing cell population, which has been maintained in culture for more than 100 passages, is referred to as TDC2 (T-ALL derived dendritic cells-2). At a later stage TDC2 cells were adapted to growth in X-VIVO 15™ medium (BioWhittaker, Walkersville, Maryland) supplemented with 2% pooled human serum (HS), and used for migration - and antigen presentation assays (Bloodbank Rivierenland, Nijmegen, The Netherlands).

Monocyte-derived DCs were generated from PBMC prepared from buffy coats as described previously (de Vries et al., Journal of Immunotherapy 2002;25:429-438; de Vries et al., Clinical Cancer Research 2003;9:5091-5100) and cultured in X-VIVO 15™ medium (BioWhittaker, Walkersville, Maryland) supplemented with 2% pooled human serum (HS) (Bloodbank Rivierenland, Nijmegen, The Netherlands). MUTZ-3 cells were cultured in 12-well tissue culture plates in Memα supplemented with 20% FCS, 10% culture supernatant of a 5637 bladder carcinoma cell line (Quentmeier et al, . Leuk.Res. 1997;21:343-350), 100 U/ml penicillin/streptomycin solution and 50µM β-mercaptoethanol. Immature DCs were obtained by culturing in the presence of IL-4 (1000u/ml), GM-CSF (100ng/ml) (both Schering-Plough International, Kenilworth, NJ) and TNFα (2.5ng/ml; Cell Genix, Freiburg, Germany) for seven days. Maturation of either TDC2 or MoDCs was induced by a 2-day incubation with LPS (2µg/ml; Sigma-Aldrich, St. Louis, MO), a combination of poly I:C (2 µg/ml; Sigma-Aldrich) and R848 (4 µg/ml; Axxora) or a cocktail of recombinant TNF-α (10 ng/ml; CellGenix), IL-1β (5 ng/ml; ImmunoTools, Friesoythe, Germany), IL-6 (15 ng/ml; CellGenix) and PGE2 (10 µg/ml; Pharmacia & Upjoin, Puurs, Belgium). MUTZ-3 derived DCs were either matured by LPS or a high dose of TNFα (75ng/ml) in accordance with earlier reports (Masterson et al., Blood 2002;100:701-703).

### Example 2: flowcytometric analysis of TDC2 cells

The phenotype of TDC2 cells and moDCs was analyzed by flow cytometry using the following primary monoclonal antibodies (mAbs): anti-HLA-ABC (W6/32), anti-HLA-DR (Q5/13) and anti-CD80 (all BD biosciences, Mountain View, CA, USA), anti-CD83 (Beckman Coulter, Mijdrecht, the Netherlands), anti-CD86, anti-mannose receptor (BD Pharmingen, San Diego, CA, USA), anti-CCR7, anti-DCIR (R&D Systems, Minneapolis, MN, USA), anti-DEC205 (eBioscience, Halle Zoersel, Belgium), anti-DC-SIGN (AZN-D1)11, anti-CLEC9A12, and anti HLA-A2.1 (clones MA2.1 and BB7.2), followed by Alexa 488-conjugated goat anti-mouse IgG (Life Technologies, Carlsbad, CA). FITC or phycoerythrin (PE)-conjugated antibodies were obtained from Becton Dickinson & CO, Franklin Lakes, NJ: CD1a, CD3, CD11b, CD11c, CD14, CD19, CD20, CD34, CD40, CD56, CD163. Appropriate isotype-matched antibodies were used as controls. Flow cytometry was performed with a FACSCalibur™ flow cytometer equipped with CellQuest software (BD Biosciences).

### Example 3: in vitro cell migration assays

To study CCR7-mediated migration, transwell inserts with 5 µm pore size polycarbonate membranes (Costar) were preincubated with 100 µl of medium in 24-well plates, each well containing 600 µl medium. 1x105 DC were seeded in the upper compartment. CCL19 (200 ng/ml, Peprotech) or CCL21 (100 ng/ml; R&D Systems) was added to the lower wells. Spontaneous migration was measured by incubation of the cells in a transwell chamber in the absence of CCL19 or CCL21. DC were allowed to migrate for 60 min. in a 5% CO2, humidified incubator at 37°C. After 60 min., DC were harvested from the lower chamber and counted by flow cytometry. All conditions were tested in duplicate.

### Example 4: Mixed lymphocyte reaction MLR

The ability of the TDC2 cells to induce T cell proliferation was studied in an allogeneic proliferation assay. In short, freshly isolated allogeneic PBMCs (1x10⁵ cells) from healthy donors were incubated with increasing amounts (5/10/20x10⁵ cells) of either TDC2 or monocyte-derived DCs that were either unstimulated or matured with LPS or maturation cocktail. After 4 days of culture, 1 µCi/well of 3H-thymidine was added per well. 3H-thymidine incorporation was measured in a beta-counter after 8 hrs of labeling.

### Example 5: Antigen specific T cell activation assay.

TDC2 cells (5x10³ per well) cells, matured with different maturation cocktails for 48 hr, were loaded with either specific peptide (gp100:280-288; 10 µM) or control peptide (gp100:154-167 or tyrosinase:369-376; 10 µM) for 4 hr in round-bottom 96 well-plates. After 4 hr allogeneic T cells (5x10⁴ per well) were added, that had been electroporated with mRNA coding for the T cell receptor recognizing gp100:280-288 peptide presented in HLA-A2 (Schreibelt et al., Blood 2010;116:564-574; Schreibelt et al., Blood 2012;119:2284-2292). After overnight co-culture CD69 expression on the gp100:280-288-specific T cells was analyzed by flow cytometry using PE-conjugated mouse anti-human CD3 and FITC-conjugated mouse anti-human CD69 (both BD Pharmingen). IFNγ-production after overnight incubation was measured in the supernatant using a standard sandwich ELISA (Pierce Biotechnology).

### Example 6: Cross-presentation by TDC2 cells

To study cross-presentation by TDC2 cells, unstimulated TDC2 cells (5x10³ per well) were incubated with gp100 long peptide (gp100:272-300; 10 µM). As controls, TDC2 cells were incubated either with gp100 short peptide (gp100:280-288; 10 µM), irrelevant non-matched peptide (gp100:154-167 or tyrosinase:369-376; 10 µM) for 4 hr. Gp100 cross-presentation was assessed by co-culturing 5x10³ TDC2 cells with 5x104 allogeneic T cells expressing the gp100:280-288 T cell receptor (TCR) in the presence or absence of 2 µg/ml LPS, or 2 µg/ml poly I:C (Sigma-Aldrich) and 4 µg/ml R848 (Axxora). After overnight co-culture CD69 expression on the gp100:280-288-specific T cells was analyzed by flow cytometry using PE-conjugated mouse anti-human CD3 and FITC-conjugated mouse anti-human CD69 (both BD Pharmingen). IFNγ-production after overnight incubation was measured in the supernatant using a standard sandwich ELISA (Pierce Biotechnology).

### Example 7: Generation and phenotypic characterization of the TDC2 cell line:

PBMCs were isolated from the blood of a 2 year old boy diagnosed with T-ALL and maintained in culture for several weeks. Leukemic blasts, obtained at diagnosis, carried a (11;14)(p13;q11)translocation, which occurs in about 7% of all T-ALL cases (Hwang and Baer, Curr.Opin.lmmunol. 1995;7:659-664). After a few weeks of culturing, loosely-adherent cell clusters grew out, giving rise to a cell type clearly distinct from the original leukemic blast population. Cytogenetic analysis revealed a normal karyotype , indicating that this cell population was unrelated to the T-ALL. Consistent with these observations, we determined by flow cytometry that the T cell markers CD3, CD4, CD7, used to define the leukemic blast population, were not present on these cells (Figure 1A).

Further flow cytometric analysis identified these cells as dendritic cell-like, as indicated by the prominent expression MHC class II molecules (HLA-DR/HLA-DQ), MHC class I (HLA-ABC), DEC205, CD1a, CD11c, and the expression of costimulatory molecules CD80, CD86 and CD83. In addition, the early B cell markers CD19 and CD20 were found present on these cells. The cells also expressed the Fc receptors CD23 (FcεRII), CD32 (FcγRIII) and CD16 (FcγRII) and C type lectin receptors dectin-1 and mannose receptor, whereas DC markers BDCA1, BDCA2, BDCA3 and BDCA4 , C type lectin receptors DC-SIGN, DCIR and CLEC9A , and the macrophage marker CD163 were absent.The same cell population was recovered repeatedly, from either blood or bone marrow from this patient while in complete remission. Again, after a few weeks of culture we acquired highly proliferating cell clusters that could be maintained for at least 100 passages.ln May-Grünwald/Giemsa stained cytocentrifuge preparations, the cells were characterized as large (sometimes binucleate) cells with large cytoplasms. Plasmamembrane extensions and vacuolizations were frequently observed, a morphology indicative of DCs or macrophages (Figure 1 B).

In the culture dish, the cells grew as large aggregates with fine membrane extensions (Figure 1 C). At higher magnifications we observed that about 10% of the cells carried prominent finger-like projections, characteristic of DCs. When adhered to fibronectin/poly-I lysine-coated coverslips and stained for HLA-DR and F-actin, a typical DC morphology was revealed, evidenced by prominent actin-rich dendritic extensions while intracellular HLA-DR positive vesicles were detectable. Hence, on the basis of cell surface expression and morphological criteria, these blood-derived cells may represent a class of immortalized (poorly adherent) myeloid/conventional DCs. Table 2 shows an overview of cell surface molecules tested and expressed by TDC2 cells.

**Table 2: Primer sequences used for real-time quantitative PCR analyses of TLRs**

| **Gene** | **Forward primer 5'→3'** | **Reverse primer 5'→3'** |
|---|---|---|
| **TLR1** | CAGTGTCTGGTACACGCATGGT (SEQ ID NO: 1) | TTCAAAAACCGTGTCTGTTAAGAG (SEQ ID NO: 2) |
| **TLR2** | GAATCCTCCAATCAGGCTTCTC (SEQ ID NO: 3) | GCCCTGAGGGAATGGAGTT (SEQ ID NO: 4) |
| **TLR3** | GTTGTCATCGAATCAAATTAAAG AG (SEQ ID NO: 5) | CATTGTTCAGAAAGAGGCCAAA (SEQ ID NO: 6) |
| **TLR4** | GGCATGCCTGTGCTGAGTT (SEQ ID NO: 7) | GCTACAACAGATACTACAAGCACACT (SEQ ID NO: 8) |
| **TLR5** | TGCCTTGAAGCCTTCAGTTATG (SEQ ID NO: 9) | CCAACCACCACCATGATGAG (SEQ ID NO: 10) |
| **TLR6** | GAAGAACAACCCTTTAGGATAG C (SEQ ID NO: 11) | AGGCAAACAAAATGGAAGCTT (SEQ ID NO:12) |
| **TLR7** | TGGAAATTGCCCTCGTTGTT (SEQ ID NO: 13) | GTCAGCGCATCAAAAGCATT (SEQ ID NO: 14) |
| **TLR8** | TTATGTGTTCCAGGAACTCAGAG (SEQ ID NO: 15) | TACCCAAGTTGATAGTCGATAAG (SEQ ID NO: 16) |
| **TLR9** | TGAAGACTTCAGGCCCAACTG (SEQ ID NO: 17) | TGCACGGTCACCAGGTTGT (SEQ ID NO: 18) |
| **TLR10** | CTGATGACCAACTGCTCCAA (SEQ ID NO: 19) | AGTCTGCGGGAACCTTTCTT (SEQ ID NO:20) |

### Example 8: TDC2 cells express a broad repertoire of TLRs

Although the initiation of effective antigen-specific immunity (or tolerance to self-antigens) is a primary feature of DCs, these cells also play a central role in the control of innate immune responses to a large variety of pathogens. To do so, DCs are equipped with a broad repertoire of receptors involved in pathogen recognition, in particular Toll like receptors (TLRs) (Iwasaki and Medzhitov, Science 2010;327:291-295). To date, 10 different members of this family have been identified, and their primary function is to sense and respond to pathogen-associated molecular patters form bacteria, viruses or parasites. Stimulation TLRs, by pathogens, provides essential stimulatory signals for DC maturation and the initiation of adaptive immune responses against these pathogens. In addition TLRs can recognize endogenous ligands (self-antigens) to initiate inflammatory responses and tissue repair without triggering adaptive immune responses (reviewed by Iwasaki and Medzhitov, Science 2010;327:291-295). Although DCs express most TLRs, different DC subsets show distinct TLR expression profiles (Schreibelt et al., Cancer Immunol.Immunother. 2010;59:1573-1582). For instance, human circulating myeloid DCs express all TLRs, with the exception of TLR9. In contrast, plasmacytoid DCs express high levels of TLR7 and TLR9, intracellular receptors that are triggered by viral DNA or RNA, leading to the secretion of high amounts of Type I IFN. Based on mRNA expression, TDC2 cells resemble moDCs and myeloid DCs, however, in contrast to most myeloid DC subsets characterized to date, the TDC2 cells appear to also express TLR7 and TLR9, whereas TLR8 expression is lacking (Figure 2). Thus, the TLR expression profile of TDC2 cells is a mixture of that of both myeloid and plasmacytoid DCs.

### Example 9: TDC2 cells efficiently take up antigens

One of the defining characteristics of DCs and other antigen presenting cells is the ability to take up soluble or particulate antigens and process these for antigen presentation. This can occur via receptor-mediated endocytosis, phagocytosis or macropinocytosis (fluid phase uptake) (Lanzavecchia, Curr.Opin.lmmunol. 1996;8:348-354). Hence, we tested the ability of TDC2 cells to take up and process particulate or soluble antigens. Hereto, TDC2 cells, seeded on coverslips, were incubated for different time-points with dextran-coated iron particles (endorem) both at 37 °C and at 4°C, fixed and subjected to Prussian blue staining to allow detection of internalized iron particles. Positive cells were scored by microscopic inspection. We observed that after two hrs of incubation at 37 °C, about 50% of the cells had internalized endorem particles, while fewer than 10% of the cells stained positive after incubation at 4°C (Figure 2A). Uptake of particles was not affected by LPS stimulation (results not shown). Similarly, we determined that TDC2 cells incubated for three hrs with FITC-labelled ovalbumin (FITC-OVA) had efficiently taken up and processed these soluble antigens, to end up in a LAMP-1 positive compartment, as determined by immunofluorescence microscopy.

### Example 10: TDC2 cells effectively stimulate lymphocyte proliferation in response to activating signals

In order to determine to what extent TDC2 cells were capable of inducing lymphocyte proliferation, an allogeneic mixed lymphocyte reaction (MLR) was performed in which we compared TDC2 cells with MUTZ-3 derived DCs (M3DCs) as well as monocyte-derived DCs (MoDCs). Freshly isolated allogeneic peripheral blood mononuclear cells (PBMCs) were isolated from two healthy donors and incubated with TDC2 cells in various effector/responder ratios. For these experiments, cells were either left unstimulated or primed with either LPS or a cytokine cocktail. Already in the absence of maturation stimuli, the TDC2 cells were able to induce some lymphocyte proliferation, consistent with relatively high expression of MHC and costimulatory molecules on these cells (Figure 3). However, lymphocyte proliferation was enhanced a further five fold after 48 hr exposure to either LPS or the cytokine cocktail, indicating that these maturation stimuli trigger a robust activation of the antigen presentation machinery in these cells. Only after incubation with the maturation cocktail, effective stimulation of lymphocyte proliferation was seen with the MoDCs, while only a moderate effect was observed with the M3DCs. These results demonstrate that TDC2 cells show strong allostimulatory capacity, which can be further enhanced in response to exposure with LPS or proinflammatory cytokines. In some experiments, unstimulated TDC2 cells already induced strong T cell activation, which was not further enhanced by TLR- or cytokine stimulation. These differences are likely due to differences in basal activation state of the TDC2 cells between experiments

### Example 11: TDC2 cells can present tumor specific antigens to CD8+ T cells

The large majority of tumor specific peptides available to the clinic to monitor tumor antigen-specific cytotoxic T cell responses, are presented in the context of HLA-A2. Although the full complement of MHC class I and class II molecules expressed on TDC2 cells has not been analyzed, we determined by flow cytometry using anti-HLA-A2 monoclonal antibodies, BB7.2 and MA2.1, that TDC2 cells are HLA-A2 positive. This allowed us to monitor the induction of (tumor) antigen-specific T cell responses by TDC2 cells. To monitor peptide-specific lymphocyte activation, PBLs expressing a specific T cell receptor (TCR) which recognizes an antigenic peptide derived from the melanoma specific antigen glycoprotein 100, (gp100:280-288) in the context of HLA-A2.1, were generated as described earlier (Schreibelt et al., Blood 2010;116:564-574). HLA-A2.1 positive TDC2 cells were either left untreated, stimulated with TLR-agonists or a cocktail of proinflammatory cytokines for 48 hrs prior to peptide loading. Subsequently, TDC2 cells were loaded with the gp100:280-288 short peptide, which can directly bind to extracellular membrane-bound HLA-A2.1. As a negative control, TDC2 cells were incubated with an irrelevant peptide (tyrosinase). After peptide-loading, TDC2 cells were co-cultured overnight with allogeneic HLA-A2.1 negative PBLs, made to express the T cell receptor that recognizes the HLA-A2.1-binding gp100:280-288 peptide. TDC2 cells induced gp100:280-288 peptide-specific T cells activation, as demonstrated by CD69 expression and IFNγ production after overnight co-incubation of TDC2 cells and gp100-specfic T cells. Interestingly, T cells activated by poly(I:C) and R848-stimulated TDC2 cells produced higher levels of IFNγ than T cells activated by unstimulated TDC2 cells, suggesting that these TLR ligands induce TDC2 activation. These data show that TDC2 cells can activate CD8+ T cells in an antigen-specific manner.

### Example 12: TDC2 cells show efficient cross presentation

DCs can present antigens to CD8+ T cells either by endogenous processing of antigens (for instance after viral infection) or by the uptake and presentation of exogenous antigens, a phenomenon known as cross-presentation. While both modes of antigen presentation are probably important for mounting an immune response to infectious diseases, in the case of cancer, the generation of a CD8+ T cells response to intact tumor antigens (for instance derived from apoptotic or necrotic cells), is dependent on efficient cross-presentation (Melief, Immunity.2008;29:372-383). To date, very few DC model cell lines were shown to be capable of effective cross-presentation. We tested the ability of TDC2 cells to cross-present extracellular antigens to the gp100 specific T-lymphocytes. To this end, unstimulated TDC2 cells were incubated with either an irrelevant peptide (tyrosinase) or a gp100-derived long peptide (gp100:273-300) that requires active uptake and processing before it can be loaded onto HLA-A2.1. As a positive control, TDC2 cells were also loaded with the gp100:280-288 short peptide, which can directly bind to extracellular membrane-bound HLA-A2.1. After peptide incubation, TDC2 cells were either left untreated or stimulated with the TLR-4 ligand LPS or a combination of the TLR-3 agonist poly I:C and the TLR7/8 agonist R848 and cocultured overnight with the gp100:280-288 specific PBLs. TDC2 cells loaded with soluble gp100 long peptide induced significant T cell activation in terms of expression of the early activation marker CD69 and production of IFNγ, indicating that TDC2 cells efficiently cross-presented gp100 peptides to CD8+ T cells. However, prior stimulation of the TDC2 cells with the TLR ligands poly(I:C) and R848 markedly increased IFNγ production by gp100-specific T cells. We conclude from these experiments that TDC2 cells can effectively cross-present and that crosspresentation can be further enhanced by TLR stimulation.

### Example 13: Activated TDC2 cells show CCL19- and CCL21-dependent migration

The ability of DCs to migrate to the lymph nodes is an essential requirement for the induction of T cell-based immunity. This migration is dependent on the chemokine receptor CCR7, highly expressed on mature DCs, and the expression of the CCR7 ligands CCL19 and CCL21, which direct homing to the lymph nodes. Consistent with the mature nature of the TDC2 cells, expression of CCR7 was readily detected on TDC2 cells and not significantly affected by further stimulation with LPS or the cytokine cocktail (Figure 4A). In line with CCR7 expression, TDC2 cells migrated in a CCL21-dependent manner in a transwell chemotaxis assay, which was not further enhanced by stimulation with LPS or cytokines (Figure 4B). However, in some experiments increased migration was observed after overnight stimulation with either LPS or cytokines, which may be caused by differences in basal activation state of the cells. Similar results were obtained with CCL19 . We conclude from these experiments that TDC2 cells express functional CCR7, which enables the cells to respond to CCL19- or CCL21-induced chemotaxis.

## Claims

1. Human cell or cell line as deposited at the DSMZ under accession number DSM ACC3168.

2. Human cell or cell line according to claim 1, with the phenotype: CD3-, CD4-, CD7-, CD163-, CD14-, CD34-, CD56-, CD19+, CD79-, CD11b-, CD11C+, CD1a+, CD40+, CD80+, CD83+, CD86+, HLA-DR+, HLA-DQ+, HLA-ABC+, DC-SIGN-, CD206-, DEC205+, DECTIN-, BDCA2-.

3. Human cell or cell line according to claims 1 or 2 wherein at least one additional gene is introduced encoding an immunotherapeutic agent

4. Mature dendritic cells obtainable by exposing the human cells or cell line according to claims 1 - 3 to a maturation stimulus.

5. Human cell or cell line obtainable by exposing the human cell or cell line according to claims 1 -4 to an antigen and allowing the cell to process the antigen.

6. Pharmaceutical composition or a vaccine comprising the human cell or cell line of claims 1 -5 and a suitable excipient.

7. Composition comprising the human cell or cell line of claims 1 - 5 for use as a medicament.

8. Composition comprising the human cell or cell line of claims 1 - 5 for use in the treatment or prophylaxis of an immune disease or cancer.

9. Composition for use according to claim 8 wherein the immune disease is selected from the group consisting of inflammatory diseases, autoimmune diseases, transplant rejections, and infectious diseases.

10. A method for stimulating T-cells in vitro wherein the human cells or cell line according to claims 1 - 5.are exposed to T-cells.

11. A method according to claim 10 wherein the T cells are tumor antigen specific T cells.

12. A method for identifying compounds that activate or inhibit a human dendritic cell function wherein the human cells or cell line according to claims 1 - 5 is exposed to said compound and wherein functional activation or inhibition of the cell function is determined.

13. A method for modulating, activating, inhibiting or stimulating the humoral and/or cellular immune system wherein a cell or cell line according to claims 1 - 5 is administered to an individual in need of such a treatment.

14. A method for efficiently presenting an antigen to the immune system wherein the antigen is contacted with the human cells or cell line according to claims 1 - 5, incubated to allow the human cells or cell line to process the antigens and wherein the thus obtained cells are contacted with immune cells such as T cells or B cells.

15. The method of claim 14 wherein the antigens are selected from the group consisting of peptides, proteins, lipids, lipopeptides, lipoproteins, carbohydrates, glycolipids, glycopeptides, glycoproteins, phosphorylated proteins and phosphorylated peptides.


SEQUENCE LISTING

<110> Stichting Katholieke Universiteit

<120> A human dendritic cell line

<130> 214 EPP0

<160> 20

<170> PatentIn version 3.5

<210> 1

<211> 22

<212>  DNA
<213>  homo sapiens
<400>  1
cagtgtctgg tacacgcatg gt
22

<210>  2
<211>  24
<212>  DNA
<213>  homo sapiens
<400>  2
ttcaaaaacc gtgtctgtta agag
24

<210>  3
<211>  22
<212>  DNA
<213>  homo sapiens
<400>  3
gaatcctcca atcaggcttc tc
22

<210>  4
<211>  19
<212>  DNA
<213>  homo sapiens
<400>  4
gccctgaggg aatggagtt
19

<210>  5
<211>  25
<212>  DNA
<213>  homo sapiens
<400>  5
gttgtcatcg aatcaaatta aagag
25

<210>  6
<211>  22

<212>  DNA
<213>  homo sapiens
<400>  6
cattgttcag aaagaggcca aa
22

<210>  7
<211>  19
<212>  DNA
<213>  homo sapiens
<400>  7
ggcatgcctg tgctgagtt
19

<210>  8
<211>  26
<212>  DNA
<213>  homo sapiens
<400>  8
gctacaacag atactacaag cacact
26

<210>  9
<211>  22
<212>  DNA
<213>  homo sapiens
<400>  9
tgccttgaag ccttcagtta tg
22

<210>  10
<211>  20
<212>  DNA
<213>  homo sapiens
<400>  10
ccaaccacca ccatgatgag
20

<210>  11
<211>  23

<212> DNA
<213> homo sapiens
<400> 11
gaagaacaac cctttaggat agc
23

<210> 12
<211> 21
<212> DNA
<213> homo sapiens
<400> 12
aggcaaacaa aatggaagct t
21

<210> 13
<211> 20
<212> DNA
<213> homo sapiens
<400> 13
tggaaattgc cctcgttgtt
20

<210> 14
<211> 20
<212> DNA
<213> homo sapiens
<400> 14
gtcagcgcat caaaagcatt
20

<210> 15
<211> 23
<212> DNA
<213> homo sapiens
<400> 15
ttatgtgttc caggaactca gag
23

<210> 16
<211> 23

<212>  DNA
<213>  homo sapiens
<400>  16
tacccaagtt gatagtcgat aag
23

<210>  17
<211>  21
<212>  DNA
<213>  homo sapiens
<400>  17
tgaagacttc aggcccaact g
21

<210>  18
<211>  19
<212>  DNA
<213>  homo sapiens
<400>  18
tgcacggtca ccaggttgt
19

<210>  19
<211>  20
<212>  DNA
<213>  homo sapiens
<400>  19
ctgatgacca actgctccaa
20

<210>  20
<211>  20
<212>  DNA
<213>  homo sapiens
<400>  20
agtctgcggg aacctttctt
20

SEQUENCE LISTING

<110>  Stichting Katholieke Universiteit

<120>  A human myeloid dendritic cell line

<130>  214 EPP0

<160>  20

<170>  PatentIn version 3.5

<210>  1
<211>  22
<212>  DNA
<213>  homo sapiens

<400>  1
cagtgtctgg tacacgcatg gt                                                22


<210>  2
<211>  24
<212>  DNA
<213>  homo sapiens

<400>  2
ttcaaaaacc gtgtctgtta agag                                              24


<210>  3
<211>  22
<212>  DNA
<213>  homo sapiens

<400>  3
gaatcctcca atcaggcttc tc                                                22


<210>  4
<211>  19
<212>  DNA
<213>  homo sapiens

<400>  4
gccctgaggg aatggagtt                                                    19


<210>  5
<211>  25
<212>  DNA
<213>  homo sapiens

<400>  5
gttgtcatcg aatcaaatta aagag                                             25


<210>  6
<211>  22
<212>  DNA
<213>  homo sapiens

<400>  6
cattgttcag aaagaggcca aa                                                22


<210>  7
<211>  19
<212>  DNA

<213>  homo sapiens

<400>  7
ggcatgcctg tgctgagtt                                                    19


<210>  8
<211>  26
<212>  DNA
<213>  homo sapiens

<400>  8
gctacaacag atactacaag cacact                                            26


<210>  9
<211>  22
<212>  DNA
<213>  homo sapiens

<400>  9
tgccttgaag ccttcagtta tg                                                22


<210>  10
<211>  20
<212>  DNA
<213>  homo sapiens

<400>  10
ccaaccacca ccatgatgag                                                   20


<210>  11
<211>  23
<212>  DNA
<213>  homo sapiens

<400>  11
gaagaacaac cctttaggat agc                                               23


<210>  12
<211>  21
<212>  DNA
<213>  homo sapiens

<400>  12
aggcaaacaa aatggaagct t                                                 21


<210>  13
<211>  20
<212>  DNA
<213>  homo sapiens

<400>  13
tggaaattgc cctcgttgtt                                                   20


<210>  14
<211>  20
<212>  DNA
<213>  homo sapiens

<400>  14
gtcagcgcat caaaagcatt                                                   20

<210>  15
<211>  23
<212>  DNA
<213>  homo sapiens

<400>  15
ttatgtgttc caggaactca gag                                               23


<210>  16
<211>  23
<212>  DNA
<213>  homo sapiens

<400>  16
tacccaagtt gatagtcgat aag                                               23


<210>  17
<211>  21
<212>  DNA
<213>  homo sapiens

<400>  17
tgaagacttc aggcccaact g                                                 21


<210>  18
<211>  19
<212>  DNA
<213>  homo sapiens

<400>  18
tgcacggtca ccaggttgt                                                    19


<210>  19
<211>  20
<212>  DNA
<213>  homo sapiens

<400>  19
ctgatgacca actgctccaa                                                   20


<210>  20
<211>  20
<212>  DNA
<213>  homo sapiens

<400>  20
agtctgcggg aacctttctt                                                   20